# EUROPEAN PATENT APPLICATION

(11) **EP 2 332 600 A1**
(43) Date of publication of application: **15.06.2011**
(21) Application number: 09405222.2
(22) Date of filing: 09.12.2009
(51) Int. Cl.: A61M 5/178, A61J 1/20, A61M 5/00

(54) **A method of filling a container with a liquid drug**

(71) Applicant: F.Hoffmann-La Roche AG, 4070 Basel (CH); Roche Diagnostics GmbH, 68305 Mannheim (DE)
(72) Inventor: Hänggi, Roger, 4208 Nunningen (CH); Rindlisbacher, Urs, Thörishaus (CH); Moia, Franco, 4402 Frenkendorf (CH); Müller, Marcel, 9014 St. Gallen (CH); Huwiler, Christoph, 6340 Baar (CH); Kühni, Florian, 3084 Wabern (CH); Wermelinger, Jörg, 8200 Schaffhausen (CH); Lischewski, Andreas, 8224 Löhningen (CH); Classen, Christian, 78250 Tengen (DE); Callenbach, Tilo, 8203 Schaffhausen (CH); Widmer, Louis, 9631 Ulisbach (CH)
(74) Representative: Münch, Martin Walter

(57) **Abstract**

The invention concerns a method of filling a container (1) with a liquid drug from a drug reservoir (2) comprising the steps:
a) connecting the container (1) and the drug reservoir (2) with a cannula (3) so that they communicate with each other;
b) reducing the inside volume of the container (1), thereby displacing a gaseous medium from the container (1) into the drug reservoir (2);
c) increasing the inside volume of the container (1), thereby displacing liquid drug from the drug reservoir (2) into the container (1); and
d) disconnecting the filled container (1) and the liquid drug reservoir (2).

The steps b) and c) are successively performed more than once before step d) is performed, thereby stepwise increasing the amount of liquid drug contained inside the container (1).

By means of the method according to the invention it becomes possible to significantly reduce the risk of a formation of foam and air bubbles within the liquid drug that is filled into the container.

## Description

The present invention relates to a method of filling an container with a liquid drug from a drug reservoir, to apparatuses for use in a method of filling a container with a liquid drug from a drug reservoir as well as to a system for extracting a liquid drug from a drug reservoir according to the preambles of the independent claims.

In the medical field, there often is the need to transfer liquid drugs, such as insulin, from a reservoir container which contains an amount of the liquid drug to a container which can receive a single dose of the drug or an amount of the drug which is suitable for a limited time of therapy of a patient. For example, doctors and nurses routinely draw medications from vials into syringes in order to inject the medications into patients. With the advent of patients taking greater charge of their medical care, this process is also performed by the patients themselves. For example, in the therapy of diabetes with the use of an insulin pump, the insulin ampoules used in the insulin pump often are not purchased as readily filled products but as empty ampoules which are then, by the patients themselves, filled with insulin prior to use from a vial containing an amount of insulin.

Since many of these patients suffer from movement disorders due to their disease and/or age, and thus have difficulties in safely handling standard syringe assemblies or the like, special filling systems have been developed in the prior art with the aim to make the process of filling ampoules from a vial easier and more safe.

One example for such a system is shown in EP 1 820 485 A1. This system consists of a number of empty, single use ampoules made of plastic, each of which is formed by a container and a piston arranged therein in displaceable manner and of a multi use piston rod with a thread arranged at one end thereof for being coupled to the piston of the ampoules. At the end facing away from the end where the piston rod can be coupled to the piston, each ampoule features a connector with a cannula by which the ampoule can be connected to a commercially available vial in such a manner that the contents of the ampoule and of the vial fluidically communicate with each other via the cannula and at the same time the ampoule and the vial are structurally connected with each other.

For filling one of the ampoules with insulin from a vial, the piston rod is connected with the piston of the ampoule by screwing it with its threaded end into a corresponding thread formed in the piston. After that, the piston is pulled back with the piston rod by means of which the inside of the ampoule is filled with air. After this has been done, the ampoule is coupled with the connector facing downwards to a vial. In a next step, the piston is fully pushed into the container of the ampoule by means of the piston rod so that the air contained inside the ampoule is displaced into the vial, thereby creating an overpressure in the vial. In this state, the whole arrangement is turned upside down so that the vial is now on top. Then, the piston rod is released so that, due to the overpressure in the vial, insulin flows through the connector into the ampoule, thereby displacing the piston inside it. Once the displacement of the piston due to the overpressure in the vial has stopped, the piston by means of the piston rod is further displaced until it reaches its original position, thereby drawing further insulin into the ampoule. In this state the vial, the connector and the piston rod are removed from the ampoule, which is now full of insulin and ready for use.

Even though by this system filling of ampoules from vials is clearly facilitated and the risk of accidental needle sticks is significantly reduced with regard to a filling with a conventional syringe arrangement, proper use of the system still needs some skills and experience in order to avoid formation of foam and air bubbles within the ampoule. Furthermore, in case the ampoule is to be filled with insulin from a smaller pen cartridge instead of from a vial, there is the risk that, when the piston is fully pushed into the container of the ampoule by means of the piston rod, the piston of the pen cartridge is pushed out of the cartridge and the insulin contained therein is spilled.

Another example of such a system having, with regard to the invention, a similar functionality and the same shortcomings as the before described system is disclosed in US 2002/0189712 A1.

Hence, it is the object of the invention to provide a method, apparatuses and a system which do not show or at least partially avoid the disadvantages of the above mentioned prior art.

This object is achieved by the method, the apparatuses and the system according to the independent claims.

A first aspect of the invention concerns a method of filling a container with a liquid drug, like e.g. a liquid pain killer or insulin, from a drug reservoir. The method comprises the following steps:
a) connecting the container and the drug reservoir with a cannula in such a manner that the inside of the drug reservoir and the inside of the container communicate with each other via the cannula while being hermetically sealed against the outside;
b) reducing the volume of the inside of the container, thereby displacing a gaseous medium from the inside of the container via the cannula into the inside of the drug reservoir;
c) increasing the volume of the inside of the container, thereby displacing liquid drug from the inside of the drug reservoir into the inside of the container for filling the container; and
d) disconnecting the filled container and the liquid drug reservoir.

According to the invention the steps b) and c) are successively performed more than once before step d) is performed, thereby stepwise increasing the amount of liquid drug contained inside the container before the container and the liquid drug reservoir are disconnected.

By means of the method according to the invention it becomes possible to significantly reduce the maximum pressure inside the liquid drug reservoir during the filling process of the container and to thereby reduce the risk of a formation of foam and air bubbles within the liquid drug that is filled into the container. Furthermore, in case the liquid drug reservoir is a pen cartridge, the risk that the piston of the pen cartridge is pushed out of the cartridge and that the liquid drug contained therein is spilled is significantly reduced.

The container to be filled preferably is a syringe type container, more preferably an ampoule, or a bag type container. Syringe type containers are formed by a substantially rigid container body and a piston arranged therein in displaceable manner, by means of which the volume of the inside of the container can be altered through displacing the piston. Ampoules are syringe type containers, for example, having a septum or a Luer-type connector, at their front face. Bag type containers have a collapsible container body and can have similar connectors for establishing fluidic communication to the interior of the container. The before mentioned container types are especially suitable for use in the method according to the invention.

Preferably, the method steps b) and c) are successively performed at least three times, at least five times, at least eight times or at least ten times before performing method step d). By doing so, filling of the container can be accomplished in several small steps which further helps avoiding foam and air bubble formation within the liquid drug transferred to the container and helps keep the maximum pressure in the liquid drug reservoir below critical values especially for pen cartridges, avoiding not only foam and air bubble forming but also avoiding pushing out the piston from the pen cartridge.

In order to use the full capacity of the container to be filled it is furthermore preferred that the method steps b) and c) are successively performed until the container is substantially filled up with the liquid drug, thus, until it contains its design capacity of liquid drug.

By advantage, method step d) is performed with the container having an inside volume which is identical to the inside volume it had when method step b) was commenced for the first time. This means in case of the filling of a ampoule that, when the empty ampoule is connected to the liquid drug reservoir for filling and when after filling the filled ampoule is disconnected from the liquid drug reservoir, its piston is positioned substantially in the same position within the container body.

In two alternative preferred embodiments of the method, before method step c) is performed for the first time, method step b) is performed or vice versa. Depending on which total pressure level in the filling system is acceptable, the one or the other alternative might be more preferable.

If the reduction in volume of the inside of the container which is performed in method step b), in each case is less than one half, preferably less than one fourth and even more preferably less than one tenth of the maximum volume of the inside of the container, several small filling steps result when filling the container until it contains its design volume of liquid drug, with the beneficial effects already mentioned above.

If the amount of the increase in volume of the inside of the container which is performed in method step c) in each case is identical to the amount of the reduction in volume of the inside which is performed in the preceding method step b), the pressure increase generated inside the filling system when performing method step b) is revoked in the subsequent method step c), thus, a stepwise increase of the pressure in the system is avoided. This is particularly of advantage in case the liquid drug reservoir is a pen cartridge, because a stepwise increase in filling system pressure can result in the piston of the pen cartridge being pushed out of the cartridge and the liquid drug contained therein being spilled.

In further preferred alternative embodiments of the method, the method step b) is performed more rapidly in each case than the method step c) following directly subsequently to that method step b) or vice versa. Depending on the type of liquid drug or type of container to be filled and on the dimensioning of the filing system, the one or the other alternative might be more preferable with regard to a short filling time and/or a prevention of foam or bubble formation, respectively.

Preferably, the speed of the increase in volume of the inside of the container in method step c) is substantially constant. This helps to avoid peaks in the flow velocity of the flow of liquid drug through the cannula.

Furthermore it is preferred that the volume of the inside of the container is increased in method step c) in such a manner that the flow velocity of the liquid drug in the cannula does not exceed 2 m/s, more preferably does not exceed 1.4 m/s, even more preferably does not exceed 1.0 m/s or even 0.5 m/s, and/or in such a manner that the volume of the inside of the container is increased in method step c) in such a manner that the Reynold's number of the flow of liquid drug in the cannula does not exceed a value where the flow transitions from laminar flow to turbulent flow and/or in such a manner that the volume of the inside of the container is reduced in method step b) in such a manner that the pressure in the drug reservoir does not exceed 2000 mbar, preferably 1000 mbar, more preferably 500 mbar.

All these measures help to further reduce the risk of foam formation and bubbles in the liquid drug filled into the container.

When, after performing method step d), the volume of the inside of the container in a further method step e) is alternately increased and reduced several times, gases that might be present in the liquid drug which has been filled into the container can be released.

By advantage, at least for performing the method steps b) and c), the drug reservoir is arranged above the container. By means of this, gravity forces can assist the filling process.

Preferably, the method steps b) and c) and where applicable method step e) are performed by means of a drive actuator that is controlled by electric or electronic or mechanical control means. Example for such drive actuators are electric motors in combination with gears and/or linkage arrangements, hydraulic or pneumatic drive arrangements, spring force driven mechanical actuators etc. However, it is also possible to manually perform these method steps.

Further it is preferred that a cannula is used in the method which is provided by a connector element by means of which connector element, when performing method step a), the drug reservoir and the container are also structurally connected with each others. By using such a connector providing the cannula, the mechanical as well as the fluidic connection between the reservoir and the connector can be established in an easy, uncomplicated manner. Furthermore, by providing the ends of the cannula within cavities formed in the connector, the risk that the user unintentionally comes in contact with these ends and injures himself or herself is significantly reduced.

In a preferred embodiment of the method, after performing step d), the connector element is separated from the drug reservoir and from the container and is disposed. Using a new connector for each filling process substantially reduces the risk of contamination of the liquid drug in the liquid drug reservoir and in the container.

A second aspect of the invention concerns an apparatus for use in a method of filling an ampoule with a liquid drug, preferably with insulin, from a liquid drug reservoir. Preferably, the apparatus is suitable for being used in the method according to the first aspect of the invention. The ampoule to be filled comprises a container and a piston arranged therein in displaceable manner. The apparatus according to the invention comprises a housing, a rod member for driving the piston of the ampoule and drive means for driving the rod member.

The rod member is moveable relative to the housing along its longitudinal axis and comprises coupling means for coupling it to the piston of the ampoule to be filled in such a manner that in both directions of said longitudinal axis of the rod member there exists a positive or a non-positive locking between the rod member and the piston. Suitable coupling means are, for example, threaded connections, bayonet connections, snap in connections, a magnet coupling or a frictional connection by means of a cone.

The drive means serve for moving the rod member along its longitudinal axis and are designed in such a manner that, upon an activation of the drive means, the rod member is moved back and forth along its longitudinal axis several times.

Preferably, the apparatus further comprises means for holding the container of the ampoule in such a manner that, at least while the drive means are activated, in both directions of said longitudinal axis of the rod member there exists a positive connection and/or a frictional connection between the container of the ampoule and the housing, so that a fully automated filling becomes possible.

A third aspect of the invention concerns an apparatus for use in a method of filling an ampoule, with a liquid drug, preferably with insulin, from a liquid drug reservoir. Preferably, the apparatus is suitable for use in the method according to the first aspect of the invention. The ampoule to be filled comprises a container and a piston arranged therein in displaceable manner. The apparatus according to the invention comprises a housing, a rod member for being coupled to the piston of the ampoule, means for holding the container of the ampoule and drive means for moving the means for holding the container.

The rod member is fix within the housing and comprises coupling means for coupling it to the piston of the ampoule to be filled in such a manner that in both directions of the longitudinal axis of the rod member there exists a positive or a non-positive locking between the rod member and the piston. Suitable coupling means are for example threaded connections, bayonet connections, snap in connections, a magnet coupling or a frictional connection by means of a cone.

The means for holding the container of the ampoule are moveable relative to the rod member along the longitudinal axis of the rod member and are adapted for holding the container of the ampoule in such a manner that, at least while the drive means are activated, in both directions of the longitudinal axis of the rod member there exists a positive and/or a frictional locking between the container of the ampoule and the means for holding the container.

The drive means are adapted for moving the means for holding the container along the longitudinal axis of the rod member and are designed in such a manner that, upon an activation thereof, the means for holding the container are several times moved back and forth along said longitudinal axis of said rod member.

By means of the apparatuses according to the second and third aspect of the invention it becomes possible to significantly reduce the maximum pressure inside the liquid drug reservoir during the filling process of the ampoule and to thereby reduce the risk of a formation of foam and air bubbles within the filled container. Furthermore, in case the liquid drug reservoir is a pen cartridge, the risk that the piston of the pen cartridge is pushed out of the cartridge and that the liquid drug contained therein is spilled is significantly reduced.

In a preferred embodiment of the apparatuses according to the second and the third aspect of the invention, the drive means are designed is such a way that, upon an activation thereof, they move back and forth said movable rod member (in case of an apparatus according to the second aspect of the invention) or said movable means for holding the container (in case of an apparatus according to the third aspect of the invention) along said longitudinal axis of the rod member at least three times, at least five times, at least eight times or at least ten times. By means of this, filling can take place in several small steps and the pressure within the filling system can be kept at a moderate level.

In a further preferred embodiment of the apparatuses according to the second and third aspect of the invention, the apparatus furthermore comprises means for identifying a filling level of an ampoule to be filled which, upon an identification of an entirely filled ampoule, deactivate the drive means and/or emit an acoustical and/or optical signal. By means of this, automated filling of an ampoule up to a specific filling level becomes possible.

In still a further preferred embodiment of the apparatuses according to the second and third aspect of the invention, the apparatus is designed in such a manner that, after deactivation of the drive means, said movable rod member (in case of an apparatus according to the second aspect of the invention) or said movable means for holding the container (in case of an apparatus according to the third aspect of the invention) is or are positioned in the same position relative to the housing in which it had been or they have been positioned before the activation of the drive means.

In still a further preferred embodiment of the apparatuses according to the second and third aspects of the invention, the apparatus is designed in such a manner that before the activation of the drive means, said movable rod member (in case of an apparatus according to the second aspect of the invention) or said movable means for holding the container (in case of an apparatus according to the third aspect of the invention) is or are positioned in a position, in which an ampoule which in the intended way is coupled to the rod member and is held in the means for holding the container has a maximum inside volume. Alternatively, the apparatus is designed in such a manner that before the activation of the drive means, said movable rod member or said movable means for holding the container is or are positioned in a position, in which an ampoule which in the intended way is coupled to the rod member and where applicable is held in the means for holding the container has, with respect to the stroke, a minimum inside volume. Depending on the total pressure level that is acceptable in the filling system, the one or the other alternative might be more preferable.

In still further preferred embodiment of the apparatuses according to the second and third aspect of the invention, the drive means are designed in such a manner that the strokes of the back and forth movement of said movable rod member (in case of an apparatus according to the second aspect of the invention) or said movable means for holding the container (in case of an apparatus according to the third aspect of the invention) are identical. This is particularly of advantage in case the liquid drug reservoir is a pen cartridge, because by means of this, a stepwise increase of the pressure inside the filling system, which could result in the piston of the pen cartridge being pushed out of the cartridge and the liquid drug contained therein being spilled, can be obviated.

In still a further preferred embodiment of the apparatuses according to the second and the third aspect of the invention, the drive means are designed in such a manner that the forth movement of said movable rod member (in case of an apparatus according to the second aspect of the invention) or said movable means for holding the container (in case of an apparatus according to the third aspect of the invention) is performed more rapidly than the back movement of same or vice versa. Depending on the type of liquid drug or the type of container to be filled and on the dimensioning of the filing system, the one or the other alternative might be more preferable with regard to a short filling time and/or a prevention of foam or bubble formation, respectively.

In a still further preferred embodiment of the apparatuses according to the second and third aspect of the invention, the drive means are designed in such a manner that the speed of the back movement of said movable rod member (in case of an apparatus according to the second aspect of the invention) or said movable means for holding the container (in case of an apparatus according to the third aspect of the invention) is substantially constant. This helps to avoid peaks in the flow velocity of the flow of liquid drug from the liquid drug reservoir to the ampoule and is of particular relevance if the fluidic connection is established by a cannula.

In still a further preferred embodiment of the apparatuses according to the second and the third aspect of the invention, the drive means comprise a drive actuator that is controlled by electric, electronic or mechanical control means. Example for such drive actuators are electric motors in combination with gears and/or linkage arrangements, hydraulic or pneumatic drive arrangements, spring force driven mechanical actuators etc.

A fourth aspect of the invention concerns a system for extracting a liquid drug, preferably insulin, from a drug reservoir. Preferably, the system is suitable for use in the method according to the first aspect of the invention. According to the invention, the system comprises an apparatus according to the second or the third aspect of the invention and an ampoule comprising a container and a piston arranged therein in displaceable manner. The ampoule is especially adapted to be coupled by its piston to the coupling means of the rod member and, where applicable, to be held or to be held and moved, respectively, by the means for holding the container. With such a system, the filling of ampoules with a liquid drug from a liquid drug reservoir can be accomplished in a safe and convenient manner.

In a preferred embodiment of the system, the ampoule and the drive means of the apparatus are designed in such a way that the amount by which the volume of the inside of the ampoule is reduced when its piston is displaced inside its container by the stroke of the forth movement performed by said movable rod member (in case of an apparatus according to the second aspect of the invention) or said movable means for holding the container (in case of an apparatus according to the third aspect of the invention) in each case is less than one half, in particular less than one fourth, in particular less than one tenth of the maximum volume of the inside of the ampoule. By means of this, filling can be accomplished with several small filling steps and it becomes possible to keep the maximum pressure in the filling system considerably low. This in turn helps to keep the pressure in the liquid drug reservoir below critical values for pen cartridges and, in case the ampoule and the liquid drug reservoir are fluidically interconnected via a cannula, helps avoiding foam and air bubble formation within the liquid drug filled into the container.

In a further preferred embodiment of the system, the system further comprises a drug reservoir containing a liquid drug and a connector with a cannula for connecting the inside of the ampoule with the inside of the liquid drug reservoir for transferring liquid drug from the drug reservoir to the ampoule. By means of this, connecting the ampoule with the liquid drug reservoir is made safe and easy.

In this embodiment of the system it is preferred that the drug reservoir, the connector and the drive means of the apparatus are designed in such a way that, when the drive means are activated, the maximum flow velocity of the flow of liquid drug in the cannula does not exceed 2.0 m/s, preferably does not exceed 1.4 m/s, even more preferably does not exceed 1.0 m/s or even 0.5 m/s.

Furthermore, it is preferred that the ampoule, the drug reservoir, the connector and the drive means of the apparatus are designed in such a way that, when the drive means are activated, the Reynold's number of the flow of liquid drug in the cannula does not exceed a value where the flow transitions from laminar flow to turbulent flow.

Also it is preferred that the ampoule, the drug reservoir, the connector and the drive means of the apparatus are designed in such a way that, when the drive means are activated, the maximum pressure in the drug reservoir does not exceed 2000 mbar, preferably does not exceed 1000 mbar and more preferably does not exceed 500 mbar.

All these measures help to further reduce the risk of foam formation and bubbles in the liquid drug that is filled into the ampoule.

Furthermore, it is preferred that the apparatus of the system further comprises means for detecting when the drug reservoir and the ampoule are disconnected from each other after a transfer of liquid drug from the drug reservoir to the ampoule while the ampoule is still coupled to the rod member and, where applicable, is still held by the means for holding the container of the ampoule and wherein the apparatus is designed in such a manner that, upon detecting such a situation, the drive means are activated in such a manner that said rod member or, in case of movable means for holding the container, said means for holding the container is or are moved back and forth several times, so that the liquid drug contained in the ampoule can be made free from gas that might be present therein.

Further preferred embodiments of the invention become apparent from the dependent claims and from the following description by way of the drawings. Therein show:
Fig. 1 a side view of an ampoule filling system of the prior art;
Figures 2a and 2b a schematic illustration of an arrangement for performing the invention;
Fig. 3 a schematic illustration of a method according to the claims;
Figures 4a to 4c illustrations with several section cut away of a first apparatus according to the invention;
Figures 5a and 5b schematic illustrations of a second apparatus according to the invention; and
Figures 6a and 6b typical courses of system pressures and the Reynold's number in the cannula during performance of a method according to the invention with the apparatus according to the Figures 4a to 4c and 5a to 5b, respectively.

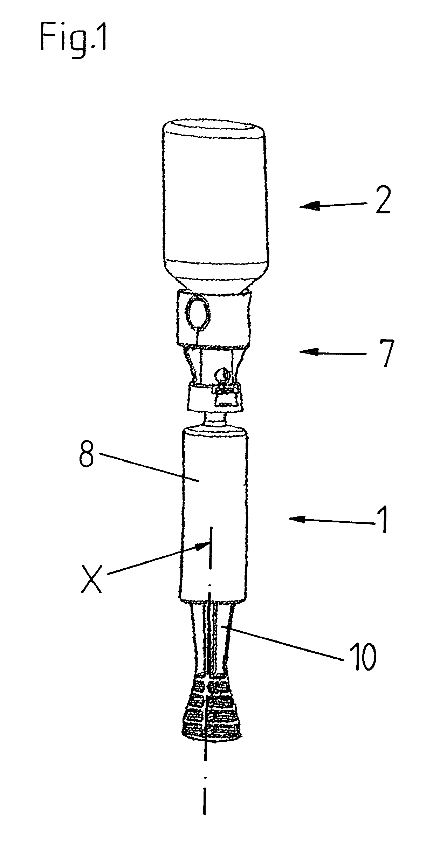

Fig. 1 shows a side view of a commercially available ampoule system. The system of Fig. 1 consists of a single use ampoule 1 made of plastic, which is formed by a container 8 and a piston (not visible) arranged therein in displaceable manner. At the backside of the ampoule, a multi use piston rod 10 is coupled to the piston by a threaded connection. At its front face, the ampoule 1 is coupled by means of a connector 7 with a vial 2 containing insulin in such a manner that the interior of the ampoule 1 and of the vial 2 fluidically communicate with each other via a cannula 3 provided by the connector 7 and at the same time the ampoule 1 and the vial 2 are structurally connected with each other. The conventional use of this system has already into detail be described in the introductory part of the description and therefore is not repeated at this point.

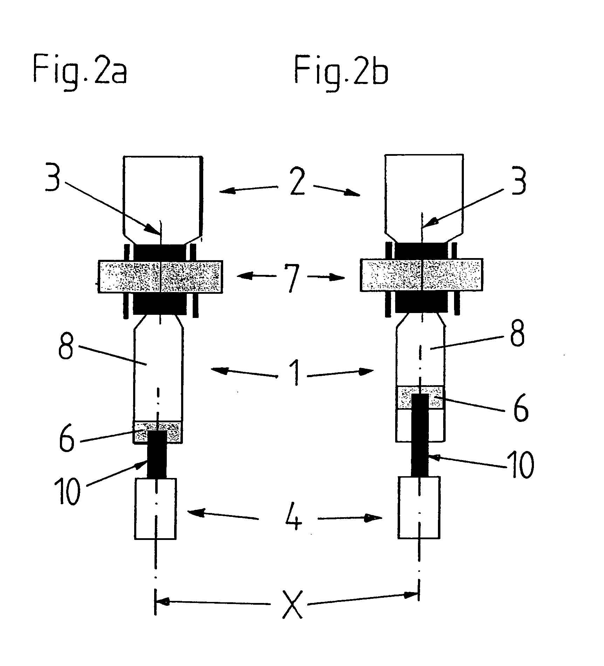

The Figures 2a and 2b show a schematic illustration of an arrangement for performing the invention. As can be seen in these Figures, the arrangement comprises an ampoule 1 which is formed by a container 8 and a piston 6 arranged therein in displaceable manner. The piston 6 is coupled via a piston rod 10 with an electrical actuator 4 by which the piston rod 10 and therewith the piston 6 can alternately be moved forward and backward along the longitudinal axis X of the piston rod 10, thereby alternately reducing and increasing the inside volume of the ampoule 1. At its front face, the ampoule is mechanically as well as fluidically coupled with a vial 2 containing insulin via a connector 7. For establishing the fluidic connection between the inside of the ampoule 1 and the inside of the vial 2, the connector 7 comprises a cannula 3 which pierces septums (not visible) at the front faces of the ampoule 1 and of the vial 2.

For performing the method according to the invention, the piston 6 by means of the actuator 4 and the piston rod 10 is several times moved back and forth between the fully retracted position shown in Fig. 2a and the partially pushed in position shown in Fig. 2b.

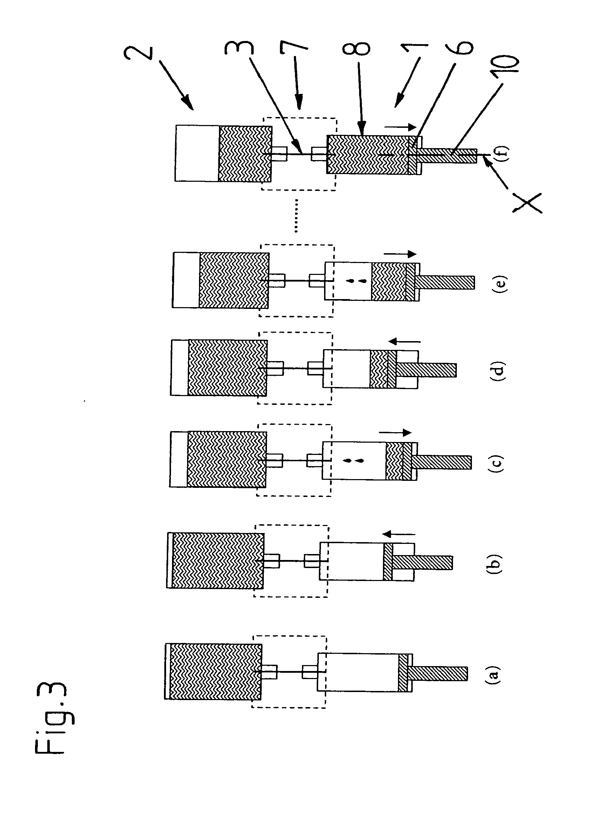

This filling process is schematically illustrated in Fig. 3, which from the left to the right shows different situations (a) to (f) during the course of filling of the ampoule, starting with situation (a) which corresponds to the situation of Fig. 2a with an empty ampoule 1. As can be seen, by moving forth and back the piston 6 several times, the ampoule 1 is stepwise filled with insulin from the vial 2, until it is substantially filled (situation f). In Fig. 3, the reference numerals for the sake of manageability have only once been applied to the illustrations (only to the situation (f)), but they are in the same fashion also applicable to the other illustrated situations (a) to (e), of course.

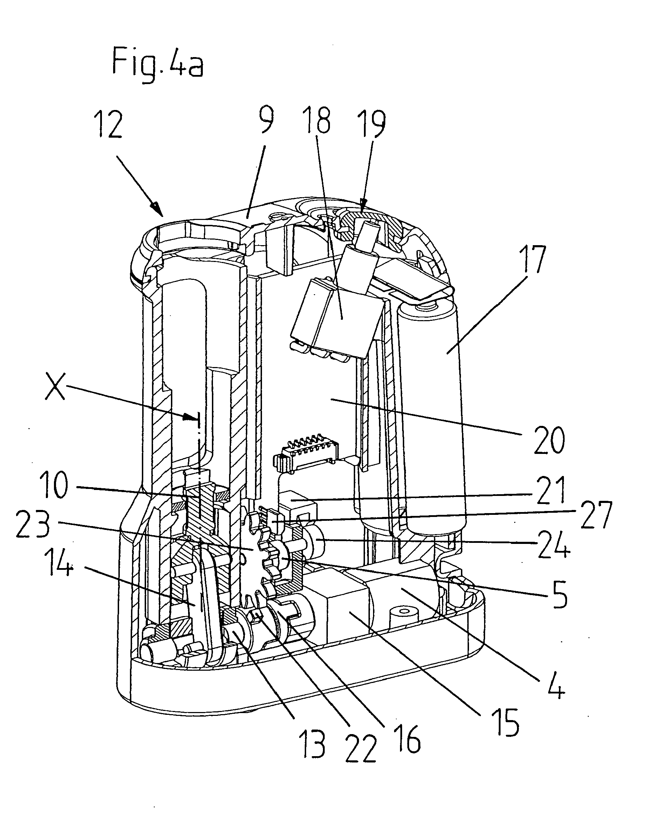

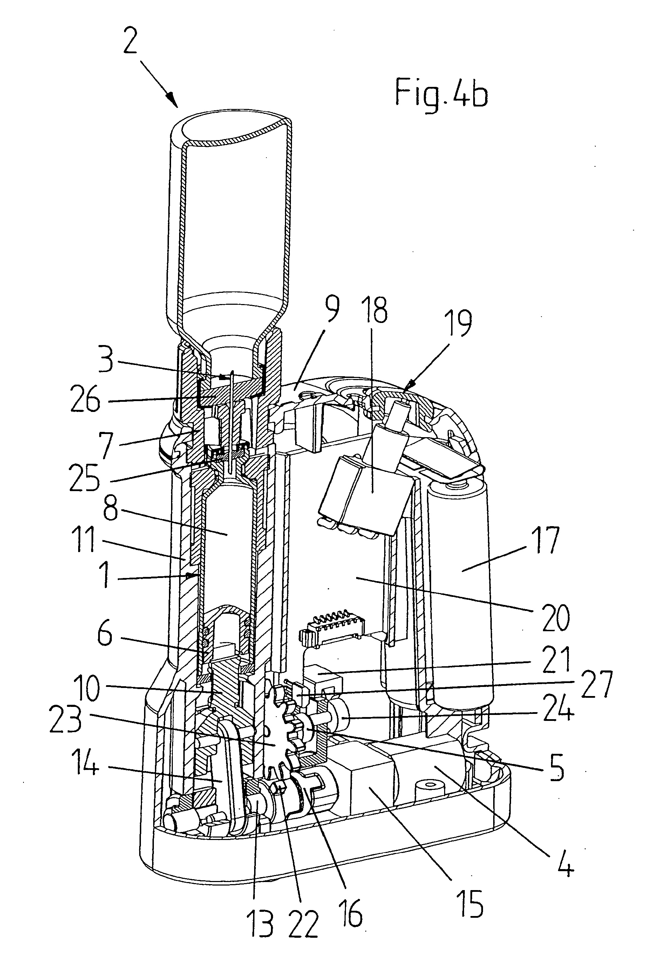

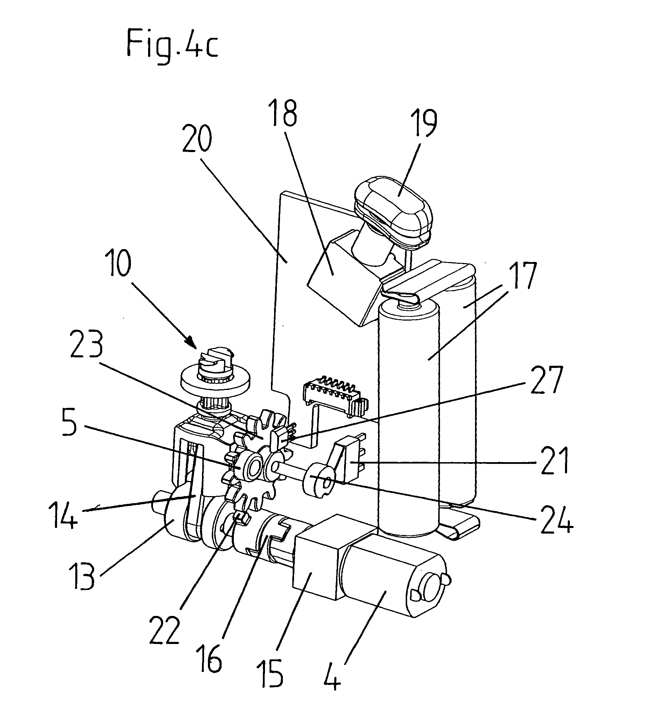

The Figures 4a to 4c show illustrations with several sections cut away of a first apparatus according to the invention. Fig. 4a shows the apparatus as such, Fig. 4b shows the apparatus in use and Fig. 4c shows the drive arrangement of the apparatus.

As can be seen, the apparatus comprises a housing 9 providing a cavity 12 for receiving the ampoule 1 to be filled. At the bottom of this cavity 12, a piston rod 10 is provided which can be moved up and down by means of a crank mechanism formed by a crankshaft 13 and a connecting rod 14 which can be moved by an electric drive unit. The electric drive unit comprises an electric motor 4 with a gearbox 15, which via an Oldham-coupling 16 is rotatably connected to the crankshaft 13. The drive unit furthermore comprises two batteries 17 for providing the electric energy for the electric motor 4 as well as an electric or electronic, respectively, control system for controlling the motor 4. This control system in turn comprises an activating switch 18 which can be actuated by pressing a button 19 at the outside of the housing 9 and a mechanical counting switch 21, which is actuated every thirteen revolution of the crankshaft 13 by a counting gear arrangement formed by a pusher dog 22 on the Oldham-coupling which drives a counting pinion 23 which in turn rotates a cam disk 24 that actuates the mechanical counting switch 21. Alternatively to the mechanical counting switch 21, there is shown a sensor 27 with a printed circuit board 20, which sensor 27 is triggered once a magnetic element 5 is positioned in front of it.

As can be seen in Fig. 4b, for the intended use of the apparatus, the ampoule 1 to be filled, which comprises a container body 8 and a piston 6 arranged therein in a displaceable manner, is inserted into the cavity 12 in the housing and is thereby radially guided by the boundary walls 11 of the cavity 12. When introducing the ampoule 1 into the cavity 12, its piston 6 is coupled to the piston rod 10 of the apparatus by rotating the ampoule 1 when it has reached the bottom of the cavity 12, thereby establishing a bayonet joint between corresponding bayonet joint mating halves formed by the piston 6 and the piston rod 10. After that, a connector 7 with a cannula 3 is mounted to the opening of the cavity 12 by a bayonet joint, thereby penetrating with the cannula a septum 25 arranged at the front face of the ampoule 1 and axially securing the ampoule 1 in the cavity 12 by positively locking it between the bottom of the cavity 12 and the connector 7. After this, the vial 2 containing insulin is pushed top down into a receiving cavity of the connector 7, thereby piercing its septum 26 with the other end of the cannula 3 and thus establishing a fluidic connection between the inner spaces of the ampoule 1 and the vial 2. This situation is depicted in Fig. 4b.

In order to achieve a maximum filling of the ampoule 1, at the start of the filling operation the piston 6 of the ampoule 1 has to be positioned in the fully retracted position. The filling operation is started by pressing the button 19, which in turn actuates the activating switch 18. Now the batteries 17 supply electrical energy to the motor 4, which via the gearbox 15, the Oldham-coupling 16 and the crankshaft 13 drives the connecting rod 14. The connecting rod 14 is pivotably connected with the piston rod 10, so that a stroke movement of the piston rod 10 results, i.e. the rotation of the axis of the motor 4 is transformed into a translatory movement of the piston rod 10 along its longitudinal axis X and therewith of the piston 6. Each upward movement of the piston 6 displaces some air from the ampoule 1 into the vial 2, and each downward movement of the piston 6 displaces some insulin from the vial 2 into the ampoule 1. The number of strokes is determined mainly by the ratio of the stroke to the length of the ampoule 1. By means of this operating principle, an overfilling of the ampoule 1 is avoided. In the present example, 10 to 15 strokes are sufficient for completely filling the ampoule 1. In order to achieve a maximum filling volume it is important to make sure that the piston at the end of the filling process again is in the fully retracted position. This is ensured by the counting switches in combination with the counting gear arrangement described above more into detail. Every complete stroke of the piston 6, the Oldham coupling 16 is rotated a full revolution (360°) so that the pusher dog 22 arranged thereon rotates the counting pinion 23 further by one tooth. After a full revolution of the counting pinion 23 the cam disk 24 actuates the counting switch 21 or alternatively the magnetic element 5 triggers the sensor 27 with the circuit board 20, which in turn stops the filling process. The number of teeth in this construction determines the number of strokes that are performed during one filling process. After the filling process has been stopped, the vial 2 as well as the ampoule 1 can be removed with the connector 7 out of the cavity 12 of the apparatus.

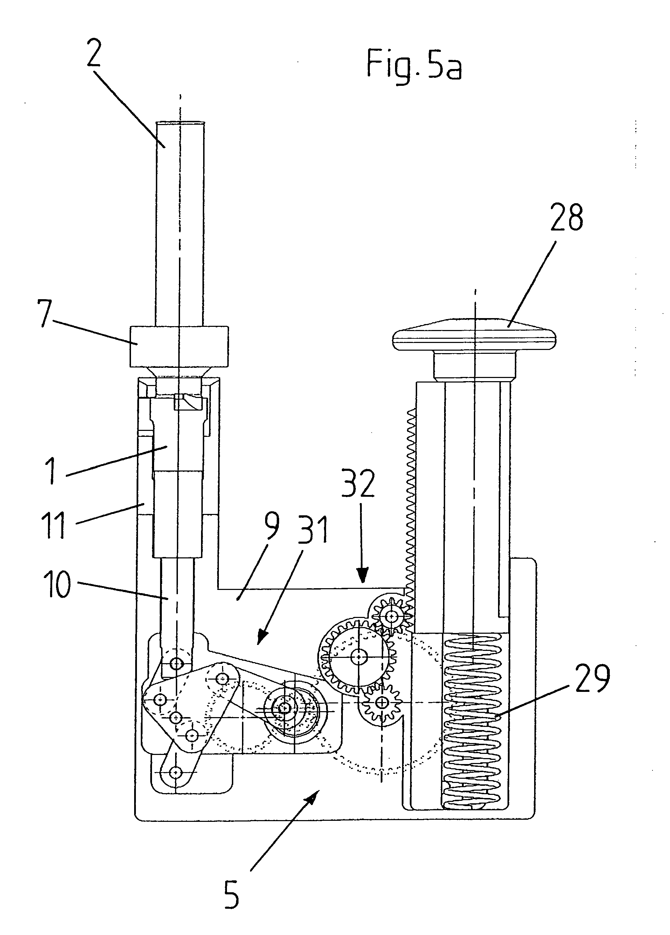

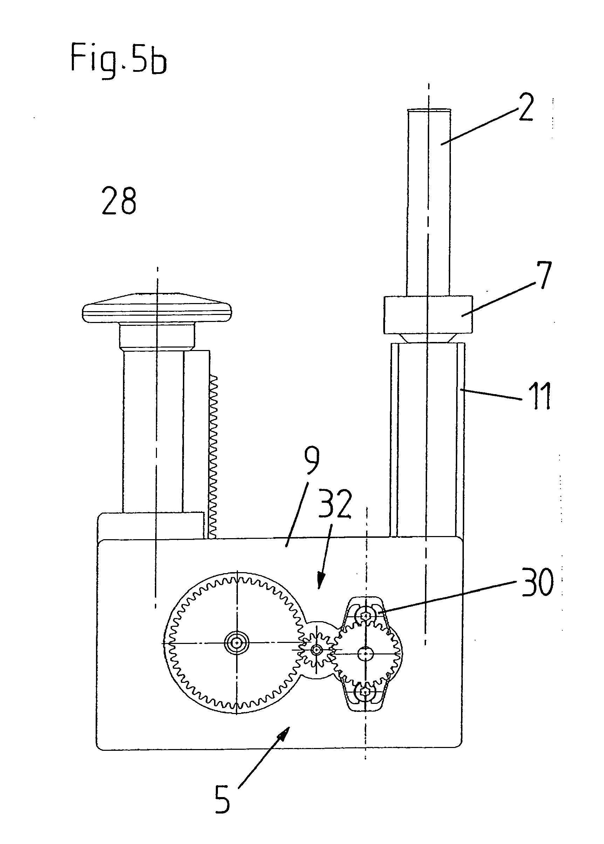

The Figures 5a and 5b show schematic illustrations of a second apparatus according to the invention, namely a front side view thereof (Fig. 5a) and a back side view (Fig. 5b). This apparatus differs from the apparatus shown in the Figures 4a to 4c mainly in that it comprises a mechanical drive unit instead of an electrical drive unit. The interconnection between the piston rod 10 and the piston (not visible) of the ampoule 1 as well as between the ampoule 1 and the reservoir 2 via the connector 7 is established in a similar manner as already described with reference to the Figures 4a to 4c. However, in the present case, the insulin reservoir 2 is not a vial but a pen cartridge 2.

For operating this apparatus, by means of pushing down the actuating button 28 a spring 29 is put under compressive stress. A commonly used mechanism makes it possible to push down the button 28 without thereby starting the filling process. Only after the spring 29 has been put under maximal compressive stress, the filling process is started. The energy stored in the spring 29 is then in a controlled manner transmitted to the piston of the ampoule 1. The essential difference when using such a mechanical drive unit instead of an electric drive unit consists in the requirement to control the spring 29 during its energy delivery. A viscous damping element 30 (rotary damper) is especially suitable for this purpose. The viscous damping element ensures that the energy of the spring with the correct pace is transmitted to a knee lever arrangement 31 which transforms a rotational force provided from a gear mechanism 32 driven by the spring 29 into a translatory movement, which drives the piston rod 10 and therewith the piston in the ampoule .1 upward and downward. The apparatus is designed in such a manner that the user starts the filling process with only one actuation of the button 28. The filling process is complete once the spring 29 is relaxed and thus no more supplies energy to the filling mechanism. The filling process as such is accomplished by the upward and downward movement of the piston which has already been described before.

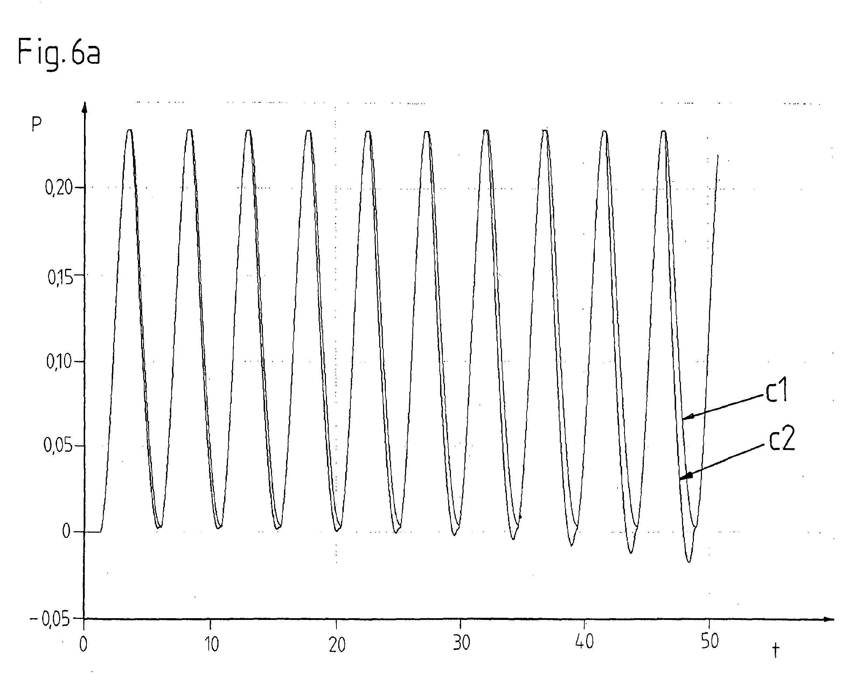

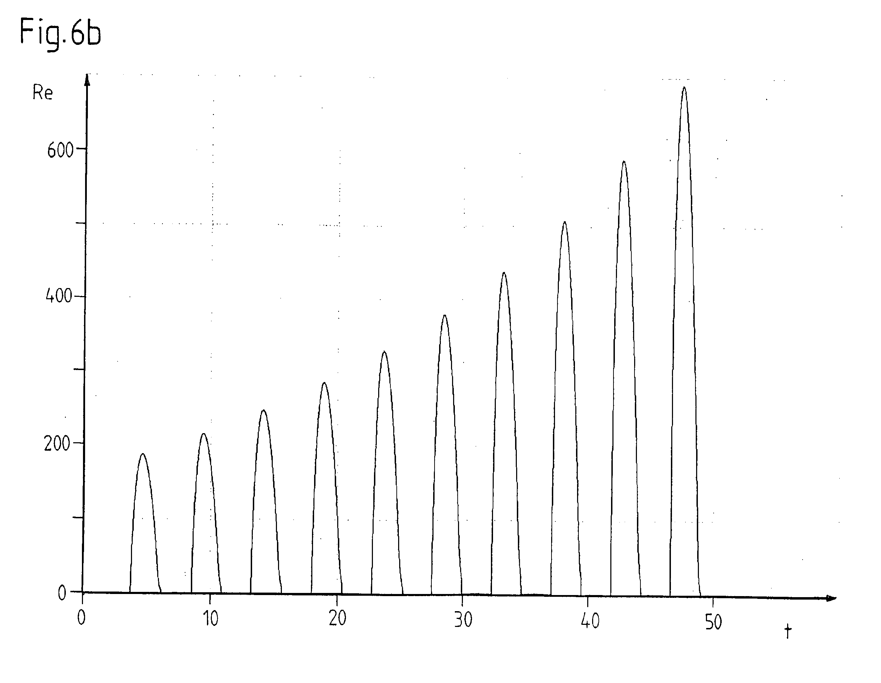

Figures 6a and 6b show typical courses of the pressure P in bar inside the pen cartridge 2 and inside the ampoule 1 over the time t in seconds as well as the Reynold's number Re of the flow of insulin in the cannula over the time t in seconds, both during performance of a method according to the invention with the apparatus according to the Figures 5a and 5b.

As can be seen in Fig. 6a, the pressure c1 inside the pen cartridge 2 which serves as insulin reservoir and the pressure c2 inside the ampoule 1 during each forward movement of the piston in the ampoule 1, in which air is displaced from the ampoule 1 into the pen cartridge 2, is increased from about 0,00 bar to about 0,25 bar. During the subsequent downward movement of the piston, in which insulin is displaced from the pen cartridge 2 via the cannula into the ampoule 1, the before mentioned pressures c1, c2 fall back to about 0.00 bar, wherein, however, with an increase of the number of strokes performed the pressure c2 inside the ampoule 1 increasingly falls slightly below 0,00 bar, as a result of the decreasing volume of air inside the ampoule 1.

As can be seen in Fig. 6b, at the same time with an increase of the number of strokes performed the maximum Reynold's number Re of the flow of insulin in the cannula, which is reached during each forward and backward movement (stroke) of the piston, increases from about 200 during the first stroke to about 700 during the last stroke, which increase is a result of the pressure c2 in the ampoule 1 increasingly falling slightly below 0,00 bar.

While there are shown and described presently preferred embodiments of the invention, it is to be distinctly understood that the invention is not limited thereto but may be otherwise variously embodied and practiced within the scope of the following claims.

## Claims

1. A method of filling a container (1) with a liquid drug, in particular insulin, from a drug reservoir (2), comprising the steps of
a) connecting the container (1) and the drug reservoir (2) with a cannula (3) in such a manner that the inside of the drug reservoir (2) and the inside of the container (1) communicate with each other via the cannula (3) while being hermetically sealed against the outside;
b) reducing the volume of the inside of the container (1), thereby displacing a gaseous medium from the inside of the container (1) via the cannula (3) into the inside of the drug reservoir (2);
c) increasing the volume of the inside of the container (1), thereby displacing liquid drug from the inside of the drug reservoir (2) into the inside of the container (1) for filling the container (1); and
d) disconnecting the filled container (1) and the liquid drug reservoir (2),
wherein before performing step d), the steps b) and c) are successively performed more than once, thereby stepwise increasing the amount of liquid drug contained inside the container (1).

2. Method according to claim 1, wherein the container (1) used in the method which is a syringe type container, in particular an ampoule (1), or a bag type container.

3. Method according to one of the preceding claims, wherein the steps b) and c) are successively performed at least three times, preferably at least eight times before performing step d).

4. Method according to one of the preceding claims, wherein the steps b) and c) are successively performed until the container (1) is substantially filled with the liquid drug.

5. Method according to one of the preceding claims, wherein step d) is performed with the container (1) having an inside volume which is identical to the inside volume it had when step b) was commenced for the first time.

6. Method according to one of the preceding claims, wherein before step c) is performed for the first time, step b) is performed or vice versa.

7. Method according to one of the preceding claims, wherein the reduction in volume of the inside of the container (1) which is performed in step b), in each case is less than one half, in particular one fourth, in particular less than one tenth of the maximum volume of the inside of the container (1).

8. Method according to one of the preceding claims, wherein the amount of the increase in volume of the inside of the container (1) which is performed in step c) in each case is identical to the amount of the reduction in volume of the inside which is performed in the preceding step b).

9. Method according to one of the preceding claims, wherein step b) is performed more rapidly in each case than the step c) following directly subsequently to that step b) or vice versa.

10. Method according to one of the preceding claims, wherein the speed of the increase in volume of the inside of the container (1) in step c) is substantially constant.

11. Method according to one of the preceding claims, wherein the volume of the inside of the container (1) is increased in step c) in such a manner that the flow velocity of the liquid drug in the cannula (3) does not exceed 2 m/s, in particular does not exceed 1.4 m/s.

12. Method according to one of the preceding claims, wherein the volume of the inside of the container (1) is increased in step c) in such a manner that the Reynold's number of the flow of liquid drug in the cannula (3) does not exceed a value where the flow transitions from laminar flow to turbulent flow.

13. Method according to one of the preceding claims, wherein the volume of the inside of the container (1) is reduced in step b) in such a manner that the pressure in the drug reservoir (2) does not exceed 2000 mbar, in particular 1000 mbar, in particular 500 mbar.

14. Method according to one of the preceding claims, wherein after performing step d), the volume of the inside of the container (1) in a further step e) is alternately increased and reduced several times, in order to free the liquid drug contained in the container (1) from gas that might be present therein.

15. Method according to one of the preceding claims, wherein at least for performing the steps b) and c), the drug reservoir (2) is arranged above the container (1).

16. Method according to one of the preceding claims, wherein the steps b) and c) and where applicable step e) are performed by means of a drive actuator (4, 5) that is controlled by electric, electronic or mechanical control means.

17. Method according to one of the preceding claims, wherein a cannula (3) is used in the method which is provided by a connector element (7) by means of which, when performing step a), the drug reservoir (2) and the container (1) are also structurally connected with each others.

18. Method according to claims 17, wherein said connector element (7), after performing step d), is separated from the drug reservoir (2) and from the container (1) and is disposed of.

19. Apparatus for use in a method of filling an ampoule (1), which is formed by a container (8) and a piston (6) arranged therein in displaceable manner, with a liquid drug, in particular insulin, from a drug reservoir (2), in particular for use in the method according to one of the preceding claims, comprising:
a housing (9);
a rod member (10) which is moveable relative to the housing (9) along a longitudinal axis (X) of the rod member (10) and which comprises coupling means for coupling the rod member (10) to the piston (6) of an ampoule (1) to be filled in such a manner that in both directions of said longitudinal axis (X) of the rod member (10) there exists a positive or a non-positive locking between the rod member (10) and the piston (6); and
drive means (4, 5) for moving the rod member (10) along said longitudinal axis (X),
wherein the drive means (4, 5) are designed in such a manner that, upon an activation thereof, the rod member (10) is moved back and forth along said longitudinal axis (X) several times.

20. Apparatus according to claim 19, further comprising means (7, 11) for holding the container (8) of the ampoule (1) in such a manner that, at least while the drive means (4, 5) are activated, in both directions of said longitudinal axis (X) of the rod member (10) there exists a positive connection and/or a frictional connection between the container (8) of the ampoule (1) and the housing (9).

21. Apparatus for use in a method of filling a container (1), which container (1) is an ampoule (1) formed by a container (8) and a piston (6) arranged therein in displaceable manner, with a liquid drug, in particular insulin, from a drug reservoir (2), in particular for use in the method according to one of the preceding claims, comprising:
a housing (9);
a rod member (10) which is fix with the housing (9) and comprises coupling means for coupling the rod member (10) to the piston (6) of an ampoule (1) to be filled in such a manner that in both directions of said longitudinal axis (X) there exists a positive or a non positive locking between the rod member (10) and the piston (6);
means for holding the container (8) of the ampoule (1) which means are moveable relative to the rod member (10) along said longitudinal axis (X) of said rod member (10) and which are adapted for holding the container (8) of the ampoule (1) in such a manner that, at least while the drive means (4, 5) are activated, in both directions of said longitudinal axis (X) of the rod member (10) there exists a positive and/or frictional locking between the container (8) of the ampoule (1) and the means for holding the container (8); and
drive means (4, 5) for moving the means for holding the container (8) along said longitudinal axis (X) of said rod member (10),
wherein the drive means (4, 5) are designed in such a manner that, upon an activation thereof, the means for holding the container (8) are several times moved back and forth along said longitudinal axis (X) of said rod member (10).

22. Apparatus according to one of the claims 19 to 21, wherein the drive means (4, 5) are designed is such a way that, upon an activation thereof, they move back and forth said rod member (10) or, in case of movable means for holding the container (8), said means for holding the container (8) along said longitudinal axis (X) of the rod member (10) at least three times, in particular at least eight times.

23. A system for extracting a liquid drug, in particular insulin, from a drug reservoir (2), in particular for use in the method according to one of the claims 1 to 18, comprising an apparatus according to one of the claims 19 to 22 and an ampoule (1) formed by a container (8) and a piston (6) arranged therein in displaceable manner, which ampoule (1) is especially adapted to be coupled with its piston (6) to the coupling means of the rod member (10) and, where applicable, to be held by the means (7, 11) for holding the container (8).
